(19) Europäisches Patentamt

European Patent Office

Office européen des brevets

(11) **EP 0 800 374 B2**

(12) **NEW EUROPEAN PATENT SPECIFICATION**

(45) Date of publication and mention
of the opposition decision:
**10.08.2005 Bulletin 2005/32**

(45) Mention of the grant of the patent:
**10.04.2002 Bulletin 2002/15**

(21) Application number: **95931555.7**

(22) Date of filing: **21.08.1995**

(51) Int Cl.⁷: **A61F 13/26**, B31B 17/00,
B31F 1/00

(86) International application number:
**PCT/US1995/010658**

(87) International publication number:
**WO 1996/020682 (11.07.1996 Gazette 1996/31)**

(54) **AN APPLICATOR FOR HOLDING AND DISPENSING A SUBSTANCE**

APPLIKATOR ZUR AUFNAHME UND ABGABE EINER SUBSTANZ

APPLICATEUR DESTINE A CONTENIR ET A DISTRIBUER UNE SUBSTANCE

(84) Designated Contracting States:
**BE DE ES FR GB IT NL SE**

(30) Priority: **29.12.1994 US 366207**
**29.12.1994 US 366074**

(43) Date of publication of application:
**15.10.1997 Bulletin 1997/42**

(60) Divisional application:
**00119789.6 / 1 060 720**

(73) Proprietor: **KIMBERLY-CLARK WORLDWIDE,
INC.**
**Neenah, Wisconsin 54956 (US)**

(72) Inventors:
• **NIELSEN, Steven, James**
**Greenville, WI 54942 (US)**
• **KRUEGER, Allen, James**
**Winneconne, WI 54986 (US)**
• **RASMUSSEN, Noel, John**
**Oshkosh, WI 54901 (US)**
• **WEYENBERG, Jeffrey, Michael**
**Appleton, WI 54915 (US)**

(74) Representative:
**DIEHL GLAESER HILTL & PARTNER**
**Patentanwälte**
**Augustenstrasse 46**
**80333 München (DE)**

(56) References cited:
| | |
|---|---|
| EP-A- 0 086 869 | EP-A- 0 475 690 |
| EP-A- 0 581 136 | EP-A- 0 605 016 |
| EP-A- 0 626 254 | AU-A- 74 659 187 |
| CH-A- 186 674 | US-A- 3 065 677 |
| US-A- 3 633 469 | US-A- 4 106 396 |
| US-A- 4 375 969 | US-A- 4 412 833 |
| US-A- 4 755 164 | US-A- 4 872 933 |
| US-A- 5 153 971 | US-A- 5 267 953 |
| US-A- 5 330 421 | US-A- 5 346 468 |

EP 0 800 374 B2

**Description**

This invention relates to an applicator adapted to hold and dispense substance.

BACKGROUND OFTHE INVENTION

There are various kinds of applicators known today which can be utilized to dispense a product or substance into a body cavity or onto the skin of a human or an animal. A tampon applicator is specifically used to insert a catamenial tampon into a woman's vagina to absorb menstrual fluid, blood and other kinds of body fluid.

Tampon applicators are available in a variety of shapes and sizes. Some applicators are constructed of paper, paperboard or cardboard while others are made from plastic or are a laminate of two or more different materials. The applicator can be either a single member wherein the user utilizes her finger to expel the tampon or the applicator can be formed from two or more members which are telescopically assembled. A typical two piece applicator employs a hollow tube for housing the tampon and a second tube or plunger which interacts with the first tube to expel the tampon into a woman's vagina.

In using a one piece applicator, the user will normally hold and position the applicator tube approximate her vagina with her thumb and middle finger. She will then use her index finger to expel the tampon into her vagina. With the two piece applicator, the user will normally hold and position the outer tube of the applicator approximate her vagina with her thumb and middle finger. The inner tube is then pushed into the outer tube by movement of her index finger so as to expel the tampon into her vagina. After the tampon is expelled, the applicator is withdrawn and discarded. It has been found that it is much easier for a woman to control the placement of the outer tube into her vagina when a fingergrip ring is provided on either the inner and/or outer tubes.

EP-0 605 016 discloses a gripping ring formed on one end of the outer tube to provide a gripping surface for the user.

An integrally formed fingergrip ring is preferred over a non-integral fingergrip since an integrally formed ring tends to be stronger in shear strength and simplifies manufacturing of the product. The fingergrip ring should be sized and configured to provide control of the applicator to ensure comfortable insertion of a tampon or other substance into a body cavity. Up until now, it has been very difficult to mass produce integrally formed finqergrip rings on tampon applicators, especially paper applicators, having a diameter of less than about 1.25 inches (about 31.75 mm). One reason for this is that paper which has been formed into a small diameter tubular member is very susceptible to being ripped ortorn when it is subjected to a curling operation. Accordingly, the apparatuses taught in U.S. Patents 1,181,965 and 3,065,677 do not work satisfactory because the frictional forces which are developed between the paper and the curling tool will literally destroy the product.

An apparatus for forming a curl on an end of a tubular member even when the tubular member is formed from paper and has a very small diameter is disclosed. An apparatus for forming a curl on the end of the applicator is also disclosed.

SUMMARY OF THE INVENTION

Briefly, this invention relates to an applicator, especially a tampon applicator, adapted to receive and hold a dispensable product or substance.

The present invention relates to an applicator 10 comprising a hollow, thin walled member 12 adapted to hold and dispense a substance 14, said member 12 being formed from at least two separate and distinct layers and having first and second spaced apart ends 26, 28 and an outwardly extending fingergrip ring 34 integrally formed adjacentto said second end, characterized in that said two layers include an inner layer 24 and an outer layer 22 and said inner layer 24 is capable of stretching more than said outer layer 22 so that the inner layer 24 is worked into a curl, the inner layer 24 forming the exterior surface of the fingergrip ring 34. The applicator can include first and second members. The first member is capable of holding the tubular member and has an external shoulder beyond which the first end of the tubular member extends. The second member is coaxially aligned with the first member and is engageable with the first end of the tubular member. The second member includes a pilot which is sized to be inserted into the first end of the tubular member. The second member also includes a sleeve which surrounds the pilot and cooperates with the external shoulder when the second member engages the first end of the tubular member to form a curling chamber. The second member further includes a curling element positioned between the pilot and the sleeve which is capable of contacting, the first end of the tubular member.

An apparatus which is not an embodiment of the invention for forming a curl on an end of the applicator includes first and second members. The first member is capable of holding the tubular member and has an external shoulder beyond which the first end of the tubular member extends. The second member is coaxially aligned with the first member and is engageable with the first end of the tubular member. The second member includes a pilot which is sized to be inserted into the first end of the tubular member. The second member also includes a sleeve which surrounds the pilot and cooperates with the external shoulder when the second member engages the first end of the tubular member to form a curling chamber. The second member further includes a curling element positioned between the pilot and the sleeve which is capable of contacting the first end of the tubular member. The apparatus further includes means for rotating one of the members and means for moving the second member into engagement with the tubular member. When the curling element contacts the first end of the tubular member the curl is formed thereon within the confines of the curling chamber.

The general object of this invention is to provide an applicator adapted to receive, hold and dispense a substance. An apparatus useful for forming a curl on an end of the applicator of the invention is disclosed. An apparatus for forming an inwardly or an outwardly extending curl on a tubular member is described.

Besides, an apparatus for forming a curl on an end of an applicator is described.

Furthermore, an apparatus for forming a curl on an end of a small diameter, paper tube is described.

Furthermore, an apparatus which can form an outwardly extending, integral curl on a tampon applicator which can serve as a fingergrip, furthermore, an apparatus which can form an outwardly extending, integral curl on a tampon applicator which can serve as a fingergrip ring, and an apparatus which can curl an end of a tubular member quickly and efficiently, and an apparatus which can curl an end of a tubular member in a cost effective manner and at relatively high manufacturing speeds are described.

Other objects and advantages of the present invention will become more apparent to those skilled in the art in view of the following description and the accompanying drawings.

BRIEF DESCRIPTION OFTHE DRAWINGS

Fig. 1 is a side view of an applicator.

Fig. 2 is a cross-sectional view of the applicator shown in Fig. 1 having a tampon pledget retained therein.

Fig. 3 is a partial cross-sectional view of the outer tube showing an integrally formed fingergrip ring having a "hollow c shaped" curl.

Fig. 4 is a partial cross-sectional view of the outer tube showing an integrally formed fingergrip ring having a "closed c shaped" curl.

Fig. 5 is a partial cross-sectional view of the outer tube showing an integrally formed fingergrip ring having a "corrugated c shaped" curl.

Fig. 6 is a partial cross-sectional view of the outer tube showing an integrally formed fingergrip ring having an "e shaped" curl.

Fig. 7 is a partial cross-sectional view of the outer tube showing an integrally formed fingergrip ring having a "right s shaped" curl.

Fig. 8 is a partial cross-sectional view of the outer tube showing an integrally formed fingergrip ring having a "left s shaped" curl.

Fig. 9 is a perspective view of a two piece tampon applicator having an inner tube telescopically slidable with an outer tube.

Fig. 10 is a cross-sectional view of the two piece tampon applicator shown in Fig. 9 having a tampon pledget retained in the outer tube.

Fig. 11 which is not an embodiment of the invention is a side view of an apparatus for forming an outwardly directed curl on an end of a tubular member.

Fig. 12 which is not an embodiment of the invention is a cross-sectional view of an apparatus shown in Fig. 11.

Fig. 13 which is not an embodiment of the invention is an end view of the first member taken along line 13--13 of Fig. 11.

Fig. 14 which is not an embodiment of the invention is an end view of the second member taken along line 14--14 of Fig. 1.

Fig. 15 which is not an embodiment of the invention is an enlarged view showing the interaction of the first and second members to form an outwardly extending curl on one end of the tubular member.

Fig. 16 is an enlarged view of a tubular member having an outwardly extending, integral curl formed on one end thereof using the curling element depicted in Fig. 15.

Fig. 17 which is not an embodiment of the invention is an enlarged view showing the interaction of the first and second members to form an outwardly extending curl on one end of the tubular member.

Fig. 18 is an enlarged view of a tubular member having an outwardly extending, integral curl formed on one end thereof using the curling element depicted in Fig. 17.

Fig. 19 which is not an embodiment of the invention is a cross-sectional view of an apparatus for forming an inwardly directed curl on an end of a tubular member.

Fig. 20 is an enlarged view of a tubular member having an inwardly extending, integral curl formed on one end thereof using the curling element depicted in Fig. 19.

## DETAILED DESCRIPTION OF THE PREFERRED EMBODIMENTS

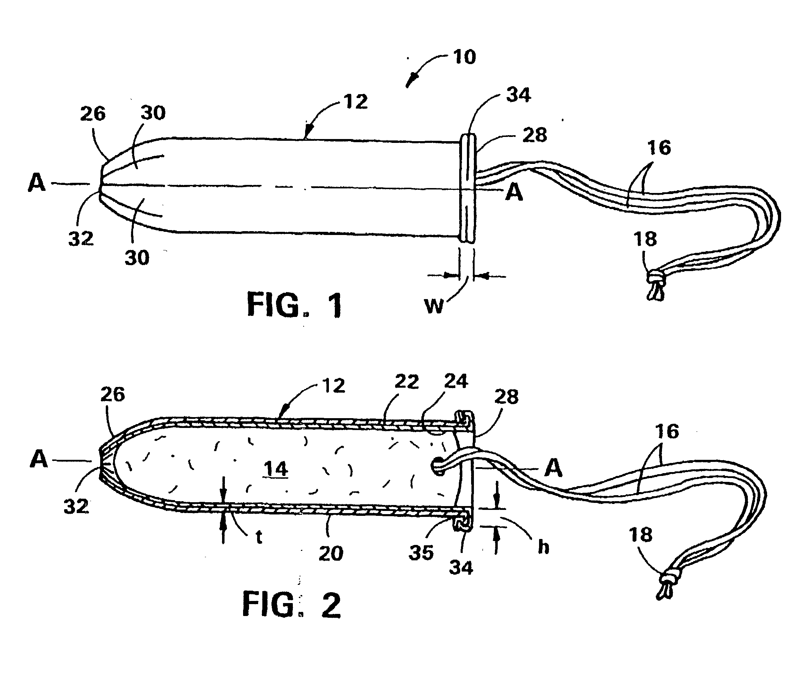

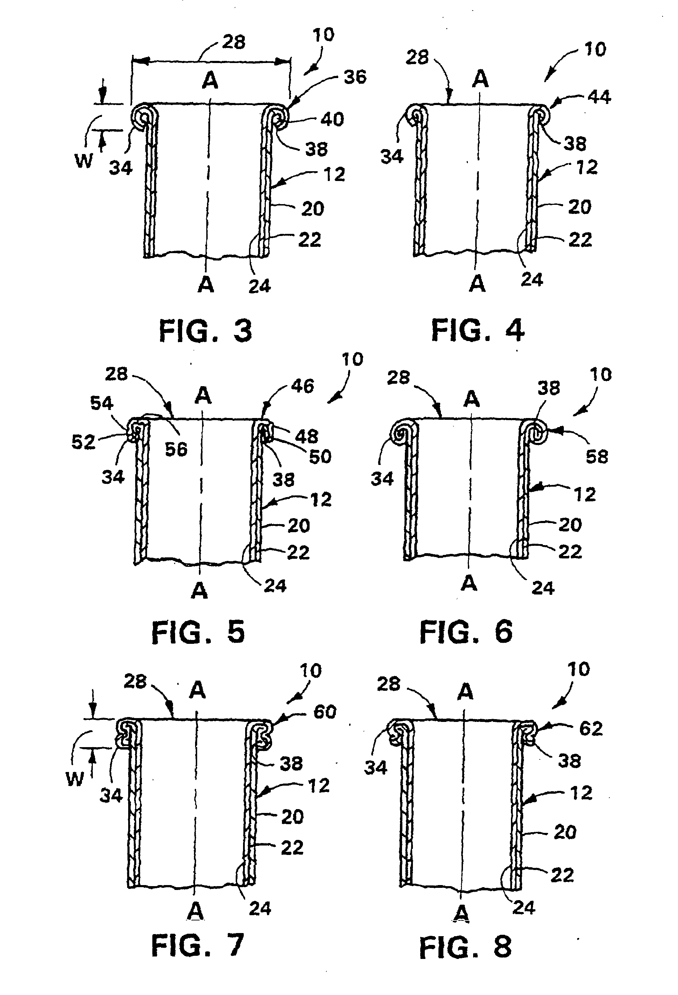

Referring to Figs. 1 and 2, an applicator 10 is shown in the form of a hollow member 12 which is designed to hold a substance 14 which can be dispensed. The applicator 10 has a relatively small diameter, less than about 1 inch (about 25.4 mm). The applicator 10 also has an overall length of between about 2 to about 12 inches (about 50.8 to about 304.8 mm) although it could be shorter or longer if desired. When the applicator 10 is used as a tampon applicator, its length should range between about 1 inch to about 3 inches (about 25.4 mm to about 76.2 mm).

The substance 14 can be almost anything, including a capsule containing medicine in a liquid, solid, granular or paste form, a medical dressing, an absorbent member, a catamenial tampon, food, etc. Although this particular invention will be described in terms of a tampon applicator 10, it should be recognized that the applicator 10 can be used to dispense a substance 14 onto the skin of a human or animal.

When the substance 14 is a tampon, it normally will include a withdrawal string 16 extending outward therefrom. The withdrawal string 16 is used to remove the soiled tampon from a woman's vagina. The withdrawal string 16 can contain a knot 18 to assure that the withdrawal string 16 does not separate from the tampon 14.

The hollow member 12 is in the shape of an elongated tube having a thin wall 20, and preferably, a circular cross-sectional profile. The wall 20 has a thickness "t" (see Fig. 2) of less than about .10 inches (about 2.54 mm), preferably less than about .03 inches (about .762 mm), and most preferably, less than about .02 inches (about 0.5 mm). The hollow member 12 is formed from at least two separate and distinct layers 22 and 24. Although only two layers 22 and 24 are depicted, the hollow member 12 could include three or more layers. The layers 22 and 24 can be formed from paper, paperboard, cardboard, plastic, thermoplastic films or any other suitable material. For .purposes of this invention, a film coating on a paper layer is considered a two layer structure.

The layers 22 and 24 can be bonded together by an adhesive such as glue, by heat, by pressure, by a combination of heat and pressure, by ultrasonics or by any other known means. Alternatively, the layers 22 and 24 can be formed as a laminate sheet and later rolled into the tubular applicator 10. It is possible to form both layers 22 and 24 out of the same material if desired. However, for economical reasons, the inner layer(s) are usually formed from a less expensive material. As mentioned above, the layers 22 and 24 are formed into a hollow, tubular member 12 having an outside diameter of less than about 1 inch (about 25.4 mm). Such a relatively small diameter can be obtained by convolutely winding, spirally winding or longitudinally seaming the material together

The material which is used to form the inner layer 24 is capable of stretching so that the inner layer 24 is worked into a curl. It is important that the inner most layer 24 is capable of stretching more than the adjacent outer layer(s) because it will form the exterior surface of the fingergrip ring 34. This means that the inner most layer 24 will be formed on a larger diameter than the outer layer 22 and if it is not capable of stretching more than the outer layer 22 it will tear or deform and thereby produce a poorly shaped fingergrip ring. The inner layer(s) 24 should be capable of being stretched at least 4 percent, preferably at least 6 percent, and most preferably, at least 8 percent more than the outer layer 22. The amount of stretch can be in either the machine direction or in the cross direction depending on how the hollow, tubular member 12 is formed.

It is also possible to coat or spray an additional layer onto the exterior layer 22 to give it a smooth or slick appearance and/or feel. The coating can decrease resistance between the applicator 10 and a body cavity, such as a woman's vagina, and aid in the comfortable insertion and removal of the applicator 10 into and out of the body cavity. It is also possible to color the different layers 22 and 24 so as to provide a visually distinctive appearance. For example, the outer layer 22 can be colored white while the inner layer 24 can be colored pink. When the two layers 22 and 24 are curled, the inner layer 24 will form the exterior surface of the fingergrip ring 34 such that the finished applicator 10 will be white with a pink fingergrip ring.

The applicator 10 also has first and second spaced apart ends, 26 and 28 respectively. The first end 26 can be shaped to facilitate comfortable insertion of the applicator 10 into a body cavity. The first end 26 can be rounded, have a semi-spherical shape, be tapered or bullet shaped. The first end 26 can also contain a plurality of independent petals 30 which are capable of flexing radially outward as the tampon 14 is expelled through the first end 26. Alternatively, the first end 26 can consist of a plurality of pleats (not shown) which can expand to provide a large opening so that the substance 14 can be expelled. The first end 26. could be completely closed but preferably contains a central opening 32 aligned along the central longitudinal axis A--A of the applicator 10. The opening 32 can be about 1/16 of an inch (about 1.587 mm) or larger and serves the purpose of limiting the force which must be exerted on the substance 14 to expel it from the hollow member 12. The lower the required expulsion force, the easier it is to comfortably insert the substance 14 into a body cavity. The presence of the central opening 32 also enables the user to visually inspect the applicator 10 to make sure a substance 14 is present to be dispensed.

Integrally formed adjacent to the second end 28 of the applicator 10 is a fingergrip ring 34. The fingergrip ring 34 extends radially outward from the central longitudinal axis A--A. The fingergrip ring 34 should have a width "w", see Fig. 1, defined as a distance measured parallel to the central longitudinal axis A--A of between about 0.02 inches to about 0.10 inches (between about 0.5 mm to about 2.54 mm) or more. Preferably, the width "w" is between about 0.05 inches to about 0.08 inches (between about 1.27 mm to about 2.03 mm). The fingergrip ring 34 also has a height "h" extending above the outer surface of the exterior layer 22 of at least about 0.1 inches (about 2.54 mm), see Fig. 2. The height of the fingergrip ring 34 can actually increase the outer diameter of the applicator 10 from between about 5 percent to about 25 percent.

It is also important that the fingergrip ring 34 have a sufficient height (h) so that it can perform it's three primary functions. In order to properly control the placement of the applicator 10 relative to the user's skin or into a body cavity, the fingergrip ring 34 should have sufficient height (h) to enable the user to feel the fingergrip ring 34 with the tips of her fingers and know where the second end 28 of the applicator 10 is located. The fingergrip ring 34 also aids in allowing the user to position the applicator 10 relative to her vagina by permitting her to pivot the applicator 10 between the tips of her thumb and middle finger. The greater the height (h) of the fingergrip ring 34, the easier it is for the user to pivot the applicator 10 in her hand. The second function is the expulsion of the tampon 14 from the hollow member 12. The fingergrip ring 34 must have a sufficient height (h) so that the user's fingertips do not slip off of the hollow member 12 when the tampon 14 is expelled out the first end 26. For good results, the outside diameter of the fingergrip ring 34 should be at least about 5 percent greater than the outside diameter of the hollow member 12. Preferably, the outside diameter of the fingergrip ring 34 should be from about 8 percent to about 20 percent greater than the outside diameter of the hollow member 12. Most preferably, the outside diameter of the fingergrip ring 34 should be from about 12 percent to about 16 percent greater than the outside diameter of the hollow member 12.

The third function of the fingergrip ring 34 is to facilitate removal of the applicator 10 away from the user's skin or away from the vagina or other body cavity. The fingergrip ring 34 should have a sufficient height (h) so that this function can be performed without having the user's fingers slip off of the applicator 10.

Referring again to Fig. 2, the fingergrip ring 34 contains a surface 35 which is essentially perpendicular to the wall 20 of the hollow member 12. This surface 35 must possess sufficient strength so that when the user's fingers exert a force on this surface 35, the first end 26 of the applicator 10 can pivot about the fingergrip ring 34. Such pivoting action facilitates positioning of the applicator 10 relative to a body cavity.

The strength and stretch characteristics of the material forming the applicator 10 will limit the maximum outside diameter of the fingergrip ring 34. The maximum outside diameter of the applicator 10 can be calculated for different kinds of material using the following formula:

$$\frac{\text{Fingergrip Ring OD - Applicator OD}}{\text{Applicator OD}} = \text{Value x 100 = X Percent}$$

where OD = outside diameter.

Using this formula for applicators constructed out of different kinds of paper, it has been established that the maximum diameter of the fingergrip ring 34 is about 120 percent of the outside diameter of the applicator 10. It should be noted that different materials will yield a different maximum diameter.

The fingergrip ring 34 must have a sufficient amount of strength to prevent the user's fingers from destroying or uncurling the fingergrip ring 34 during use. The fingergrip ring 34 will encounter three different periods in which a force will be directed on it. The three periods include a controlling period where the applicator is positioned relative to a body cavity, an expulsion period where the substance is expelled into the body cavity and a removal period where the applicator 10 is removed from the body cavity. The first force that the fingergrip ring 34 must be capable of withstanding is a squeezing force as the user positions the applicator 10 adjacent to and into her body cavity. Normally only two fingers are positioned on the fingergrip ring 34 and the force exerted on the fingergrip ring 34 is not uniformly distributed about the circumference thereof. Instead, the force impinges on the circumference of the fingergrip ring 34 at two arcuate locations which are spaced approximately 180 degrees apart. Each arcuate locations can encompass an area defined between about 45 degrees to about 110 degrees. The force during this period can range from about 50 grams to about 200 grams.

The second force that the fingergrip ring 34 experiences is during the expulsion period and this represents the greatest amount of force. This force will be directed onto the fingergrip ring 34 in a direction parallel to the longitudinal centerline A--A of the hollow member 12 as the substance is expelled into the body cavity. This force will be in an opposite direction to the direction in which the fingergrip ring 34 was curled. The expulsion force of many tampon applicators is in the range of about 250 grams to about 800 grams (about 0.55 lbs to about 1.75 lbs). Therefore, the strength of the fingergrip ring 34 should be at least about 250 grams (about 0.55 lbs), preferably, greater than about 800 grams (about 1.75 lbs), and most preferably, greater than about 1500 grams (about 3.30 lbs) so as to provide a degree of safety.

The third force that the fingergrip ring 34 must be capable of withstanding is the removal force when the applicator 10 is withdrawn from the body cavity. This force, like the controlling force, is in the range of about 50 grams to about 200 grams.

The strength of the fingergrip ring 34 or the amount of force it is capable of handling before deforming or uncurling, can be measured using a Chatillon force tester equipped with a digital force gauge. Both pieces of equipment are available from Chatillon having an office located at 7609 Business Park Drive, Greensboro, North Carolina 27409. A tension/compression tester, model TCM-200 and a Chatillon digital force instrument, model DFI work well.

The fingergrip ring 34 provides a structure which will act as a stop for the tips of a user's thumb and finger(s) thereby allowing another finger of the user or another member to push the substance 14 out through the forward or first end 26 of the applicator 10.

The amount of curl necessary to obtain the fingergrip ring 34 can vary depending upon the configuration of the fingergrip ring 34, the kind of material used to form the applicator 10, the number of layers, the thickness of the combined layers, etc. Normally, the second end 28 of the applicator 10 will be curled upon itself for at least 180 degrees. Another way of stating this is to say that the layers 22 and 24 are curled to an extent such that at least one fold is present and the material forming the curl will be aligned parallel to the layers 22 and 24 forming the tubular member 12. In some embodiments, it may be necessary or desirable to curl the material from between about 180 degrees to about 450 degrees. A curl of between about 270 degrees to about 360 degrees is necessary for certain curl configurations.

Referring to Figs. 3-8, several specific curl configurations are depicted. In Fig. 3 a "hollow c curl" 36 is shown formed on the second end 28 of the applicator 10. The "hollow c curl" 36 is obtained by curling both layers 22 and 24 approximately 270 degrees so that the distal end 38 of the tubular applicator 10 is brought into contact with the exterior surface of the first or outer layer 22. The "hollow c curl" 36 has a c-shaped cross-sectional configuration. In addition, the "hollow c curl" 36 contains a hollow center or void area 40.

In Fig. 4, an alternative "c curl" 44 is shown formed on the second end 28 of the applicator 10. The "c curl" 44 is obtained by curling both layers 22 and 24 approximately 270 degrees so that the distal end 38 of the tubular applicator 10 is brought into contact with the exterior surface of the first or outer layer 22. In addition, the two layers 22 and 24 are pressed during the formation process against the hollow, tubular member 12 such that no noticeable void area is present. This configuration has less open area than that shown in Fig. 3 but the curl 44 may still exhibit a limited void area.

Referring to Fig. 5, a "corrugated curl" 46 is shown formed on the second end 28 of the applicator 10. The "corrugated curl" 46 is obtained by curling both layers 22 and 24 approximately 270 degrees so that the distal end 38 of the tubular applicator 10 is brought into contact with the exterior surface of the first or outer layer 22. In addition, the "corrugated curl" 46 contains a hollow center or void area 48. The void area 48 gives the "corrugated curl" 46 a lesser density than the material forming the tubular wall 20. As the "corrugated curl" 46 is being formed, the material of both layers 22 and 24 is shaped into folds having alternating ridges 50 and grooves 52. The ridges 50 and grooves 52 can occur on both the outside and inside surfaces, 54 and 56 respectively, of the "corrugated curl" 46. The ridges 50 and the grooves 52 on the outside surface 54 will provide a roughened surface which is less likely to slip in a user's hand.

Referring to Fig. 6, an "e curl" 58 is shown formed on the second end 28 of the applicator 10. The "e curl" 58 has the appearance of a backward "e" cross-sectional configuration oriented parallel to the longitudinal axis A--A. The "e curl" is obtained by curling both layers 22 and 24 approximately 360 degrees so that the distal end 38 of the tubular applicator 10 is brought into parallel alignment with the outer layer 22 and is located adjacent thereto. In this particular embodiment, a void area is not present. However, depending upon the amount of compression, if any, that is applied during and/or after curling, a void area may be present along the fold lines of the "e curl." It should also be recognized that by stretching and curling the first and second layers, 22 and 24 respectively, the fingergrip ring 34 may be formed with a lesser density than the material forming the tubular wall 20.

Referring to Figs. 7 and 8, two different versions of an s-shaped curl are shown formed on the second end 28 of the applicator 10. In Fig. 7, a "right s curl" 60 is depicted while in Fig. 8, a "left s curl" 62 is shown. In the "right s curl" 60, an s-shape is obtained with the free end of the "s" being positioned adjacent to and in contact with the exterior surface of the first or outer layer 22. In the "left s curl" 62, an s-shape is obtained with the free end of the "s" being positioned away from and aligned approximately 90 degrees relative to the exterior surface of the first or outer layer 22. The right and left "s curls," 60 and 62 respectively, have an s-shaped cross-sectional configuration.

It should be noted that even though several specific curl configurations are described and shown, other curl designs are possible and may be employed without departing from the present invention.

Referring again to Figs. 3-8, the amount of material which is present in the fingergrip ring 34 is best described when locking at a cross-sectional view of the curl. When viewing the cross-sectional views, one will notice that each curl is comprised of tube wall material and usually some amount of void area 40. Given that the tube wall is made from less expensive material and is typically multilayered, it has a thickness that is somewhat substantial. This gives the applicator sufficient strength to withstand the external pressures exerted on it. The outward extending fingergrip ring 34 must also have sufficient strength and height to function properly in use. The outside diameter of the fingergrip ring 34 is substantially greater than the outside diameter of the tube and this means that the paper fibers are altered or stretched in order to occupy the larger diameter of the fingergrip ring 34. Consequently, the type of material, the thickness of the material, the amount of stretch of the material, the initial outside diameter of the tube, and the outside diameter of the fingergrip ring 34 are all interrelated. Accordingly, two terms that will be used to describe this interaction are "cross-sectional area" and "wall density" of the fingergrip ring 34.

The "cross-sectional area" of each fingergrip ring 34 is comprised of a percentage of tube material and a complementary percentage of void area 40. The cross-sectional area of each fingergrip ring 34 is comprised of from between about 70 percent to about 95 percent of material and a complementary void area of between about 5 percent to about 30 percent. Preferably, the cross-sectional area of each fingergrip ring 34 is comprised of from between about 80 percent to about 90 percent of material and a complementary void area of between about 10 percent to about 20 percent. This is a much greater percent of material in the fingergrip ring 34 than what is present in existing paper drinking cups where the amount of material present in the curl is between about 10 percent to about 20 percent and the void area is between about 80 percent to about 90 percent. The ratio of material to void area in a fingergrip ring 34 is determined by the type of material from which the ring is formed, the particular height dimension "h" needed, the required strength needed, etc. It has been found that the height and strength of the fingergrip ring 34 are crucial parameters in determining how effective the fingergrip ring 34 is in aiding control, insertion and removal to the applicator 10.

The "wall density" of each fingergrip ring 34 is determined using the standard definition of density as defined by "Engineering Materials & Their Application," 2nd edition, page 52, published by Flinn/Trojan, copyrighted 1981. Density is defined as "mass divided by volume" and is usually expressed in terms of grams per cubic centimeter or kilograms per cubic meter. In order to form a suitable fingergrip ring 34, the tubular wall 20 should have a density of between about 0.4 grams per cubic centimeters to about 1.0 grams per cubic centimeters. For a two ply paper applicator, a density of about 0.6 grams per cubic centimeters works well. The density of the wall which forms the fingergrip ring 34 is preferably less than the density of the tubular wall 20. However, an acceptable range is between about 20 percent less than the density of the tubular wall 20 to about 20 percent greater than the density of the tubular wall 20.

The fingergrip ring 34 can be formed by contacting one end of the circumference of the hollow member 12 with a rotating tool. The tool contacts the hollow member 12 and moves parallel to its longitudinal centerline A--A. As the tubular wall 20 is curled into a fingergrip ring 34, the fibers of the inner layer 24, assuming the inner layer 24 is constructed of a material like paper, are stretched and worked. This causes the fibers to be separated or become spaced out on a microscopic scale. The density of the material consequently decreases. If the fingergrip ring 34 is not subsequently compressed, it will be less dense than the hollow member 12 and may contain a void area. It is possible to compress the fingergrip ring 34 and reduce or eliminate the void area. This will cause the fingergrip ring 34 to become more dense. The fingergrip ring 34 could be compressed such that it has a higher density than the tubular wall 20 from which it was formed. However, too much compression creates an unacceptable weak fingergrip ring 34. If the density at the top of the fingergrip ring 34 is increased too much, the fingergrip ring 34 can become brittle and a weak hinge point is thereby created. This is undesirable when using of the fingergrip ring 34 on a tampon applicator 10.

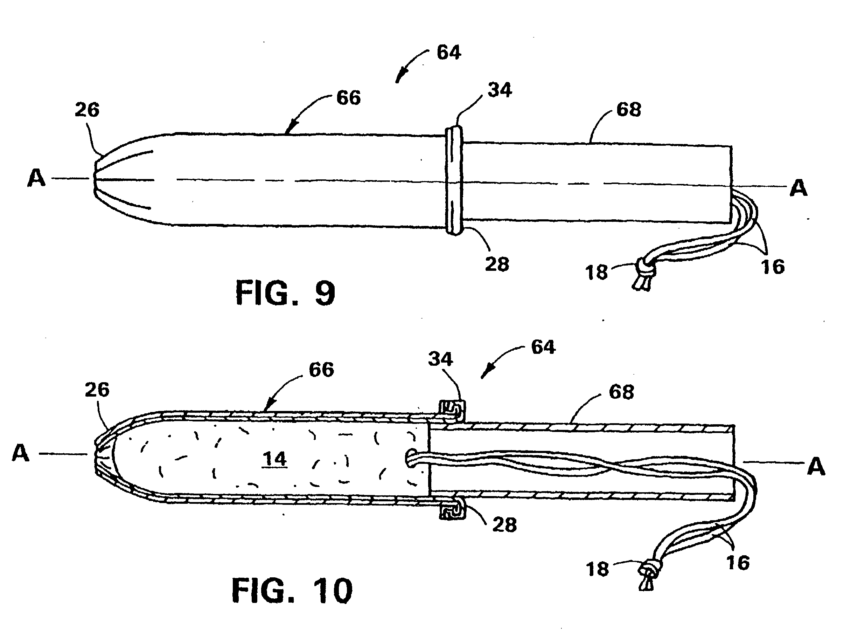

Referring now to Figs. 9 and 10, a two piece tampon applicator 64 is depicted which includes a first member 66 and a second member 68. The first member 66, or outer tube as it is sometimes referred to, is hollow and is sized and configured to receive and retain a catamenial tampon 14. The first member 66 has an outside diameter of less than about 1 inch (about 25.4 mm), preferably less than about 0.75 inches (about 19.05 mm), and most preferably, less than about .625 inches (about 15.875 mm). The first member 66 can be constructed as taught above for the hollow member 12 and should contain at least two separate-and distinct layers. Preferably, at least one of the layers is paper. The second member 68, or inner tube as it is sometimes referred to, is sized and configured to telescopically slide within the inner circumference of the first member 66. The second member 68 is preferably hollow although it does not have to be. As the second member 68 is pushed into the first member 66, the tampon 14 is expelled through the forward end 26 of the first member 66. After the tampon 14 is positioned in a woman's vagina, the applicator 64 is properly discarded.

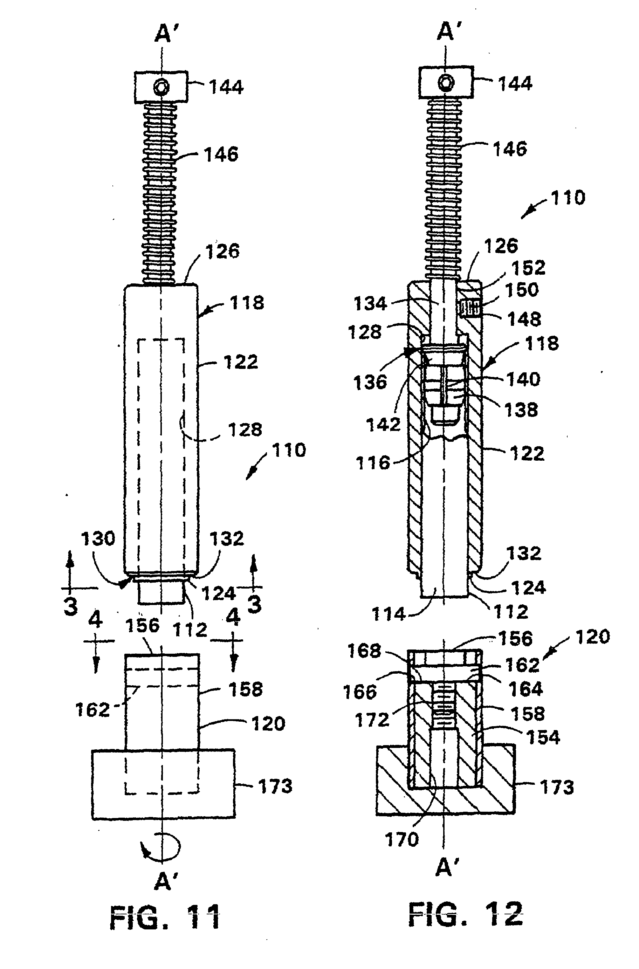

Referring to Figs. 11-14 which are not embodiments of the invention, an apparatus 110 for forming a curl on an end of a tubular member 112 is shown. By "tubular member" is meant a member having a cavity or opening 114 formed therein or therethrough with a wall 116 approximate the end which is to be curled. The wall 116 should be relatively thin. The cavity or opening 114 should extend to at least one outside surface thereof. The tubular member 112 can be cylindrical, non-cylindrical, conical, frusto-conical or of some other shape which is opened at one or more ends. The tubular member 112 does not have to have a uniform outside diameter although such a diameter is preferred for many articles.

Two articles which can be curled by the apparatus 110 include a cylindrical tube which is useful as a tampon applicator and a cylindrical tube which is useful as a medicinal applicator. A tampon applicator facilitates positioning of an absorbent tampon into a woman's vagina and a medicinal applicator can be used to dispense medication into a body cavity or onto the skin of a human or an animal. One specific application for a medicinal applicator is to dispense medication, such as a yeast infection medication, into a woman's vagina. The curl can be inwardly or outwardly extending. The outwardly extending curl can serve as a fingergrip ring to aid in holding the applicator relative to a woman's vagina.

The tubular member 112 can be of any desired length. However, when used as a tampon applicator or as a medicinal applicator, the tubular member 112 should have a length of between about 1 inch to about 112 inches (about 25.4 mm to about 305 mm) and, preferably, between about 2 inches to about 6 inches (about 50.8 mm to about 152.7 mm). The most preferred length for a tampon applicator is about 3 inches (76.2 mm) and the most preferred length for a medicinal applicator, used to dispense a yeast infection medication, is about 6 inches (about 152.4 mm).

The tubular member 112 can be constructed of any kind of material. For use as a tampon applicator or as a medicinal applicator, the tubular member 112 should be constructed of paper, paperboard, cardboard, plastic, thermoplastic film or a combination of such materials. The tubular member 112 can also be formed as a laminate consisting of at least two separate layers. Each layer can be of a similar or different material. It is also anticipated that the outer layer of the tubular member 112 can be coated with a suitable coating to give it a certain characteristic. For example, the coating can be used to give the applicator a smooth, slippery, water-permeable or water-impermeable characteristic.

If two or more layers are utilized to form the tubular member 112, they can be bonded together by an adhesive, such as glue, by heat and/or pressure, by ultrasonics or by any other known means.

The apparatus 110 which is not an embodiment of the invention for forming a curl on a tubular member 112 is especially useful when the tubular member 112 has an outside diameter of less than about 1.25 inches (about 31.7 mm), preferably, less than about 1 inch (about 25.4 mm), and most preferably, less than about 0.75 inches (about 19.05 mm). A diameter as small as about .25 inches (about 6.35 mm) can be curled using this apparatus 110. Tubular members having such relatively small diameters can be obtained by convolutely winding, spirally winding, longitudinally seaming, extruding or, plastic molding the material together. The apparatus 110 is also particularly useful in curling a tubular member 112 having a wall 116 with a thickness of less than about 0.125 inches (about 3.175 mm), preferably less than about 0.10 inches (about 2.54 mm) and, most preferably, less than about .05 inches (about 1.27 mm). Such wall thicknesses are typically used for tampon applicators and medicinal applicators, especially those constructed of paper,'paperboard, cardboard, plastic, etc.

Referring again to Figs. 11-14 which are not embodiments of the invention, the apparatus 110 includes a first member 118 and a second member 120. It is anticipated that one of the members 118 or 120 will be held stationary while the other member is rotated. For purposes of discussion, the first member 118 will be described as being held stationary while the second member 120 is rotated. However, one skilled in the art will recognize that the first member 118 can be made to rotate while the second member 120 is held stationary. Likewise, it is also possible to rotate both the first and second members, 118 and 120 respectively, at different speeds in order to form the curl. The rotational speed can vary between about 200 to about 5,000 rpm, preferably below about 2,000 rpm; and most preferably, between about 300 to about 1,800 rpm.

The first member 118 is designed to hold the tubular member 112 stationary. The first member 118 is depicted as an elongated member having a central, longitudinal axis A'--A'. The first member 118 includes a housing 122 having first and second spaced apart ends, 124 and 126 respectively. The housing 122 also has an elongated bore 128 formed therethrough which is coaxially aligned with the central, longitudinal axis A'--A'. The bore 128 extends between the first and second ends 124 and 126 of the housing 122. The bore 128 is sized and configured to be capable of receiving the tubular member 112.

Extending axially outward from the first end 124 of the housing 122 is a shoulder 130. The shoulder 130 is depicted as an external shoulder having a circular cross-sectional profile and having an outer periphery 132. The outer periphery 132 is sized to mate with a portion of the second member 120. The thickness of the shoulder 130 is relatively thin although the specific thickness will vary depending upon the thickness of curl one desires to form. When the tubular member 112 is held in the first member 118, one end of the tubular member 112 will extend beyond the shoulder 130. This end of the tubular member 112 is the end which will be curled.

The first member 118 also includes a piston rod 134 which is partially situated in the bore 128. The piston rod 134 has a piston 136 attached to an end thereof which is located in the bore 128. The piston 136 includes a sleeve 138 having an outside diameter which is less than the inside diameter of the bore 128. The sleeve 138 contains an axially aligned slot 140 formed therethrough which enables the sleeve 138 to create a large enough diameterto produce a frictional fit with the tubular member 112. The piston 136 also has a conical surface 142 positioned inward from the sleeve 138 which acts as a stop for the tubular member 112. The conical surface 142 functions to axially align the tubular member 112 in the bore 128. The tubular member 112 contacts the piston 136 and the inside surface of the bore 128. In this position, the tubular member 112 will be held stationary by the frictional contact with the sleeve 138.

The opposite end of the piston rod 134 terminates in an enlarged head 144. Surrounding the piston rod 134 between the head 144 and the second end 126 of the housing 122 is a spring 146. The purpose of the spring 146 is to bias the push rod 134 outward so that the piston 136 is positioned away from the first end 124 of the housing 122.

This position permits the tubular member 112 to be placed in the bore 128 with only a predetermined amount extending out beyond the shoulder 130. It should be mentioned that although the spring 146 is taught as the biasing means, one skilled in the art may wish to use some other biasing mechanism, such as an electric, hydraulic or pneumatic member.

After a curl has been formed on an end of the tubular member 112, the tubular member 112 can be removed from the bore 128 by pushing the piston rod 134 downward or inward into the bore 128. As the piston rod 134 is pushed downward, the spring 146 will become compressed. As the piston rod 134 and the piston 136 are moved toward the first end 124 of the housing 122, the tubular member 112 will be displaced from the bore 128. With a majority of the tubular member 112 cleared from the bore 128, the tubular member 112 can be manually removed from the sleeve 138. Once the tubular member 112 is fully removed from the piston 136, the compressed spring 146 will urge the piston rod 134 and the piston 136 back to their original position within the bore 128. The first member 118 will then be ready to accept another tubular member 112 which is to be curled.

The first member 118 further contains a threaded hole 148 aligned perpendicular to the central bore 128. The threaded hole 148 is designed to receive a set screw 150. The piston rod 134 has a flat surface 152 machined into it which extends along a major portion of it's length between the piston 136 and the enlarged head 144. The set screw 150 is threaded into the threaded hole 148 and contacts the flat surface 152. This action prevents rotation of the piston rod 134 relative to the housing 122. It should be noted that in some situations, the rotation of the piston rod 134 is not undesirable and therefore there will not be a need for the set screw 150.

The second member 120 of the apparatus 110 which is not an embodiment of the invention is coaxially aligned along the central, longitudinal axis A'--A' of the first member 118 and is movably relative to the first end 124 of the first member 118. For purposes of discussion only, the second member 120 will be described as being aligned vertically below the first member 118, although the members 118 and 120 could be positioned at some other orientation if desired.

The formation of either an outwardly directed curl or an inwardly directed curl will be dictated by the configuration of the first and second members, 118 and 120 respectively. The configuration needed on the second member 120 to form an outwardly directed curl on one end of the tubular member 112 will first be described. The configuration of the tooling needed to form an inwardly directed curl will be explained later.

The second member 120 is engageable with the end of the tubular member 112 which extends outward past the shoulder 130 when the tubular member 112 is held in the bore 128 of the first member 118. The second member 120 includes a pilot 154 which is depicted as a cylindrical member. The pilot 154 has a first end 156 of reduced outside diameter which is sized and configured to be inserted into the outwardly extending end of the tubular member 112. Preferably, the first end 156 has a circular cross-sectional profile with an outside diameter which is at least about .002 inches (about .05 mm) smaller than the inside diameter of the tubular member 112. The insertion of the first end 156 of the pilot 154 into the tubular member 112 will assure that the wall 116 of the tubular member 112 cannot curl inward as the first and second members, 118 and 120 respectively, contact one another. The first end 156 of the pilot 154 can be inserted into the tubular member 112 any desired amount. However, the first end 156 of the pilot 154 should be inserted a distance of at least about 0.125 inches (about 3.17 mm) or more into the tubular member 112.

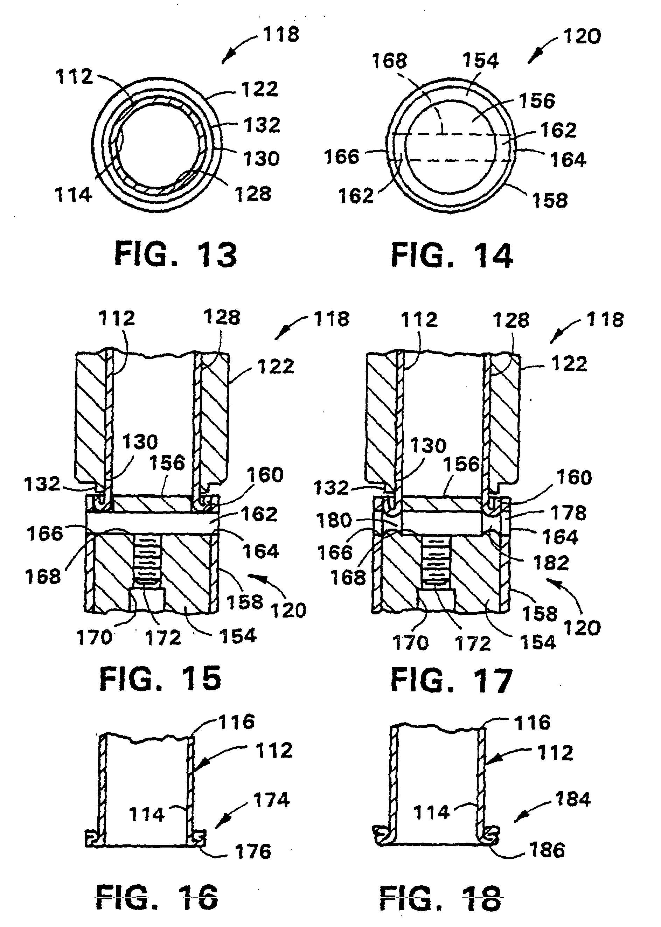

Referring to Fig. 15 which is not an embodiment of the invention, the second member 120 also includes a sleeve 158 which surrounds the pilot 154. The sleeve 158 has an inside diameter which is in contact with the outer diameter of the pilot 154 except near the first end 156. At the first end 156 of the pilot 154, the inside diameter of the sleeve 158 is spaced apart from the outer diameter of the first end 156 of the pilot 154. This clearance establishes an open area around the outer circumference of the tubular member 112 when the first and second members, 118 and 120 respectively, are moved into engagement. The open area provides a circumferential opening into which the outer end of the tubular member 112 can be curled outwardly relative to the central, longitudinal axis A'--A'.

The inner diameter of the sleeve 158 cooperates with the outer periphery 132 of the shoulder 130 to form a curling chamber 160 when the second member 120 engages an end of the tubular member 112. Preferably, the clearance between the inner diameter of the sleeve 158 and the outer periphery of the shoulder 130 is between about .001 inches to about .005 inches (about .02 mm to about .127 mm). The sleeve 158 and pilot 154 are stationary relative to one another and are designed to move axially together.

The second member 120 further includes a curling element 162 positioned through the pilot 154 and the sleeve 158 which is designed to contact the outwardly extending end of the tubular member 112 and form a curl thereon. The curling element 162 can be in the form of an elongated pin or cylindrically shaped member. The curling element 162 can be a solid or hollow member. The curling element 162 can also have a uniform outside diameter, as shown in Fig. 15, or a non-uniform outside diameter as depicted in Fig. 17. The curling element 162 is inserted through and retained in two transversely oriented apertures 164 and 166 formed in the sleeve 158 as well as a transversely oriented aperture 68 formed through the first end 156 of the pilot 154. The curling element 162 can be spaced vertically away from the first end 156 of the pilot 154 by a distance of at least about 1/16 of an inch (about 1.58 mm). The exact distance which the curling element 162 is spaced away from the first end 156 of the pilot 154 can vary.

The pilot 154 also has a central passageway 170 formed therein which is aligned along the central, longitudinal axis A'--A'. At least a portion of the passageway 170 is threaded to receive a set screw 172. The set screw 172 is designed to contact the curling element 162 and prevent it from rotating. It has been found that a better looking and more functional curl can be obtained when the curling element 162 is held stationary. However, for some applications, one may find that the curling element 162 can move or rotate without affecting the final appearance of the finished curl.

The second member 120 is designed to be inserted into a rotatable chuck 173 and can be rotated at a predetermined speed. The speed can vary depending upon the type of material the tubular member 112 is constructed of. The second member 120 is rotated while the first and second members, 118 and 120 respectively, are moved axially relative to one another. The first and second members, 118 and 120 respectively, can be reciprocally mounted relative to one another and timed to produce an automated setup. The speed at which the first and second members, 118 and 120 respectively, will engage can be controlled. It should be noted that the first and second members, 118 and 120 respectively, will approach one another and slidably contact each other without actual abutment. Actual contact and engagement occurs between the second member 120 and the end of the tubular member 112. The engagement of the first and second members, 118 and 120 respectively, causes the curling element 162 to contact the end of the tubular member 112 and form a curl thereon. The curl will be limited to the confines of the curling chamber 160.

The curling element 162 is designed to contact the exposed circumference of the tubular member 112. When the tubular member 112 is a cylindrical tube, the circumference is a complete 360° as depicted in Fig. 13. The curling element 162, see Fig. 14, is designed to contact this circumferential surface at two separate and distinct locations which are preferably approximately 180° apart. This arrangement permits the curling element 162 to contact less than about 50°, preferably less than 40°, and most preferably, less than about 30° of the circumference of the tubular member 112 at any time. This is important because this feature minimizes the buildup of frictional energy and allows a small diameter tubular member 112 to be curled without tearing or destroying the material from which it is constructed. If the curling element 162 contacted the entire circumference of the tubular member 112 simultaneously, frictional forces would prevent the formation of an acceptable curl having a good visual appearance. It has been found that the higher frictional forces also tend to generate additional heat which can hinder the formation of a good quality curl.

It should be mentioned that the curling element 162 can be coated, hardened or plated to enhance its useful life and to form a better quality curl. One coating process which has provided good results is Magnaplate HMF™ which is a proprietary multi-step coating process that creates an extremely hard, mirror-smooth surface with a low coefficient of friction. The Magnaplate HMF™ process using an electroless nickel coating, which has been modified by adding polymers, work fine. The Magnaplate coating process is available from General Magnaplate Corp. having an office at 1331 Route 1, Linden, New Jersey 07036.

The first and second members, 118 and 120 respectively, can be moved into cooperation with one another such that the second member 120 exerts a force on the end of the tubular member 112. This force can vary but it has been from experimentation that a force of approximately .5 to about 10 pounds is sufficient to curl a tubular member 112 formed from paper, paperboard, cardboard, etc. A greater force may be needed to form a curl on a tubular member constructed of plastic. Preferably, a force of less than about 5 pounds'is adequate for curling an end of a small diameter paper tube, and most preferably, a force of about 3 pounds is sufficient.

Referring to Fig. 16, the tubular member 112 is shown having an outwardly extending, integral curl 174 formed on one end thereof. This curl 174 is obtained by using the tooling depicted in Fig. 15. The curl 174 is referred to as an "s" curl having a square corner 176 because of it's shape. It has been found that when the curling element 162 has a uniform outer diameter, that the square corners are obtained.

Referring to Fig. 17 which is not an embodiment of the invention, the first and second members, 118 and 120 respectively, are similar to those depicted in Fig. 15 exceptthatthe curling element 162 is replaced by a curling element 178 having a non-uniform outer diameter. The curling element 178 is an elongated, cylindrical pin having two spaced apart grooves 180 and 182 formed therein. Each groove 180 and 182 has a smaller outside diameter than the outside diameter of the remainder of the curling element 178. The grooves 180 and 182 are situated between the first end 156 of the pilot 154 and the sleeve 158. The grooves 180 and 182 are vertically aligned with the circumferential end of the tubular member 112 as well as with the radially outwardly situated curling chamber 160. The cross-sectional profile of each groove 180 and 182, depicted in Fig. 17, shows that each has a concave profile. Other cross-sectional profiles can also be utilized. These other cross-sectional profiles include square, rectangular, convex, semi-spherical, etc.

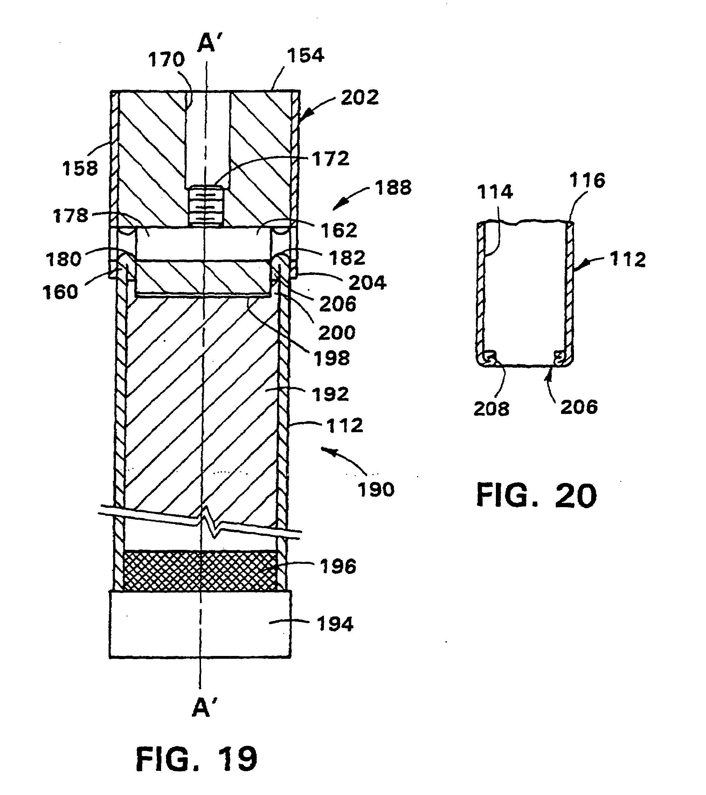

Referring to Fig. 18, the tubular member 112 is shown having an outwardly extending, integral curl 184 formed on one end thereof. This curl 184 is obtained by using the tooling depicted in Fig. 17. The curl 184 is referred to as an "s" curl having a rounded corner 186 because of it's shape. It has been found that when the curling element 178 has a non-uniform outer diameter, that the rounded corners 186 are obtained.

Referring to Fig. 19 which is not an embodiment of the invention, an apparatus 188 is shown for forming an inwardly directed, integral curl on an end of a tubular member 112. The apparatus includes a first member 190 which is capable of holding the tubular member 112 stationary. The first member 190 has an elongated central shaft 192 extending axially outward from an enlarged base 194. The diameter of the shaft 192 is sized to closely match the inside diameter of the tubular member 112 which is to be curled. The shaft 192 is of sufficient length to allow the tubular member 112 to be inserted over it and held in a desired position. The shaft also has a knurled section 196 which will assist in holding the tubular member 112 stationary and limit rotation movement. The shaft 192 further has a first end 198 surrounded'by an external shoulder 200. The shoulder 200 extends axially beyond the first end 198 and the tubular member 112 extends axially beyond the external shoulder 200 when fully inserted onto the shaft 192. The amount which the tubular member 112 extends beyond the shoulder is predetermined depending upon how much curl is intended to be formed on the end thereof.

The apparatus 188 also includes a second member 202 which is coaxially aligned with and engageable with the first member 190. The second member 202 is constructed of the same parts as the second member 120, explained above. The only difference, other than size dimension, is that in the second member 202, the sleeve 158 has a surface 204 which is not aligned flush with the first end 156 of the pilot 154. This aspect allows the first end 198 of the shaft 192 to engage the first end 156 of the pilot 154. When this occurs, the outer periphery of the first end 156 of the pilot 154 engages the inner periphery of the shoulder 200 and the curling chamber 160 is formed between the grooves 180 and 182 and the inner surface of the sleeve 158. The curling chamber 160 permits the end of the tubular member 112 to have an inwardly directed curl 206 to be formed thereon as the first and second members, 190 and 202 respectively, engage one another.

It has been found that when forming the inwardly directed curl 206, that a better quality curl can be obtained when the curling element 178, having a non-uniform diameter, is used. However, the material from which the tubular member 112 is constructed, it's wall thickness, it's diameter, the type of curl, etc. all contribute the final quality of the formed curl.

As stated above, either the first or second members, 190 and 202 respectively, can be rotated and either can be reciprocated toward and away from the other member. Furthermore, the apparatus 188 can include removal means for removing the tubular member 112 from the shaft 192. The removal means can be similar to that described and depicted above in Figs. 11 and 12 which are not embodiments of the invention or it can be some other mechanism, such as a pneumatic, hydraulic, electrical or electro-mechanical mechanism.

Referring to Fig. 20, the tubular member 112 is shown having an inwardly extending, integral curl 206 formed on one end thereof. This curl 206 is obtained by using the tooling depicted in Fig. 19. The curl 206 is referred to as an "s" curl having a rounded corner 208 because of its shape. It has been found that when the curling element 178 has a non-uniform outer diameter, that the rounded corners 208 are obtained.

It should also be mentioned that formation of the curls 174 and 184 can be enhanced by adding some moisture to the paper tube. The addition of as little as .05 percent moisture is beneficial. Also, the addition of a wax to the end of the tubular member 112 which is to be curled will enhance the finished curl. Lastly, it is possible to heat set the curl 174 and 184 for a predetermined period of time to assure that it retains its shape and to increase its ability to keep its shape under load. A heat set in the range of about 93°C (200°F) to about 204°C (400°F) for a time period of between about 1 second to about 10 seconds works well. An adequate heat set for a curl integrally formed on a tubular member constructed of at least two layers of paper can be obtained by heating the curl to about 149°C (300°F) for about 3 seconds. Any temperature below the burn melting temperature of the material from which the tubular member 112 is constructed may work provided the time period is adjusted to prevent destruction of the tubular member 112 itself.

While the invention has been described in conjunction with several specific embodiments, it is to be understood that many alternatives, modifications and variations will be apparent to those skilled in the art in light of the aforegoing description.

Accordingly, this invention is intended to embrace all such alternatives, modifications and variations which fall within the scope of the appended claims.

**Claims**

1. An applicator (10) comprising a hollow, thin walled member (12) adapted to hold and dispense a substance (14), said member (12) being formed from at least two separate and distinct layers and having first and second spaced apart ends (26, 28) and an outwardly extending fingergrip ring (34) integrally formed adjacent to said second end, **characterized in that** said two layers include an inner layer (24) and an outer layer (22) and said inner layer (24) is capable of stretching more than said outer layer (22) so that the inner layer (24) is worked into a curl, the inner layer (24) forming the exterior surface of the fingergrip ring (34).

2. The applicator (10) of claim 1 wherein said fingergrip ring (34) has a density less than that of said walled member (12).

3. The applicator (10) of claim 2 wherein said fingergrip ring (34) has a density which is up to about 20 percent less than the density of said walled member.

4. The applicator (10) of claim 1 wherein said fingergrip ring (34) is comprised of between about 70 percent to about 95 percent material and between about 5 percent to about 30 percent void area.

5. The applicator (10) of claim 1 wherein said fingergrip ring (34) is comprised of between about 80 percent to about 90 percent material and between about 10 percent to about 20 percent void area.

6. The applicator (10) of claim 1 wherein said fingergrip ring (34) is formed by curling said second end (28) of said applicator (10) upon itself for at least 180 degrees.

7. The applicator (10) of claim 6 wherein said fingergrip ring (34) is formed by curling said second end (28) of said applicator (10) upon itself for at least 270 degrees.

8. The applicator (10) of claim 6, wherein said fingergrip ring (34) is formed by curling said second end (28) of said applicator (10) upon itself for about 360 degrees.

9. The applicator (10) of claim 6 wherein said member (12) has a central longitudinal axis and wherein said fingergrip ring (34) is capable of withstanding a force of at least 250 grams applied thereon at a direction parallel to said central longitudinal axis.

10. The applicator (10) of claim 9 wherein said thin walled member (12) contains an inner layer (24) and an outer layer (22) and said inner layer (24) is capable of stretching at least about 4 percent more than said outer layer (22).

11. The applicator (10) of claim 10 wherein said inner layer (24) is bonded to said outer layer (22) by an adhesive.

12. The applicator (10) of claim 9 wherein said fingergrip ring (34) is comprised of between about 70 percent to about 95 percent material and between about 5 percent to about 30 percent void area.

13. The applicator (10) of claim 9 wherein said fingergrip ring (34) is comprised of between about 80 percent to about 90 percent material and between about 10 percent to about 20 percent void area.

14. The applicator (10) of claim 9 wherein said fingergrip ring (34) has a c-shaped cross-sectional configuration.

15. The applicator (10) of claim 9 wherein said fingergrip ring (34) has an e-shaped cross-sectional configuration.

16. The applicator (10) of claim 9 wherein said fingergrip ring (34) has an s-shaped cross-sectional configuration.

17. The applicator (10) of claim 1 wherein the member is a first member (66) adapted to hold and dispense a tampon, and wherein the applicator (10) comprises additionally a second member (68) telescopically slidable within said first member (66), said second member (68) adapted to expel said tampon as it is moved into said first member (66).

18. The applicator (10) of claim 17 wherein said first member (66) has an outside diameter of less than about 2.54 cm (about 1 inch).

19. The applicator (10) of claim 18 wherein said first member (66) has an outside diameter of less than about 1.9 cm (about 0.75 inches).

20. The applicator (10) of claim 17 wherein said first member (66) is formed from at least three separate and distinct layers.

21. The applicator (10) of claim 17 wherein one of said layers is paper.


**Patentansprüche**

1. Applikator (10) umfassend ein hohles, dünnwandiges Element (12), das vorgesehen ist, um eine Substanz (14) zu halten und abzugeben, wobei das Element (12) aus wenigstens zwei separaten und unterschiedlichen Lagen gebildet ist und erste und zweite beabstandete Enden (26, 28) und einen sich nach außen erstreckenden Fingergriffring (34) aufweist, der benachbart zu dem zweiten Ende integriert gebildet ist,

**dadurch gekennzeichnet, dass**

die zwei Lagen eine innere Lage (24) und eine äußere Lage (22) umfassen und die innere Lage (24) befähigt ist, sich mehr als die äußere Lage (22) zu dehnen, so dass die innere Lage (24) zu einer Einrollung verarbeitet ist, wobei die innere Lage (24) die äußere Oberfläche des Fingergriffrings (34) bildet.

2. Applikator (10) gemäß Anspruch 1, wobei der Fingergriffring (34) eine Dichte aufweist, die geringer ist als die des wandigen Elements (12).

3. Applikator (10) gemäß Anspruch 2, wobei der Fingergriffring (34) eine Dichte aufweist, die bis zu etwa 20 Prozent geringer ist als die Dichte des wandigen Elements.

4. Applikator (10) gemäß Anspruch 1, wobei der Fingergriffring (34) aus zwischen etwa 70 Prozent und etwa 95 Prozent Material und aus zwischen etwa 5 Prozent und etwa 30 Prozent Hohlraumfläche besteht.

5. Applikator (10) gemäß Anspruch 1, wobei der Fingergriffring (34) aus zwischen etwa 80 Prozent und etwa 90 Prozent Material und aus zwischen etwa 10 Prozent und etwa 20 Prozent Hohlraumfläche besteht.

6. Applikator (10) gemäß Anspruch 1, wobei der Fingergriffring (34) durch Einrollen des zweiten Endes (28) des Applikators (10) um wenigstens 180 Grad gebildet ist.

7. Applikator (10) gemäß Anspruch 6, wobei der Fingergriffring (34) durch Einrollen des zweiten Endes (28) des Applikators (10) um wenigstens 270 Grad gebildet ist.

8. Applikator (10) gemäß Anspruch 6, wobei der Fingergriffring (34) durch Einrollen des zweiten Endes (28) des Applikators (10) um etwa 360 Grad gebildet ist.

9. Applikator (10) gemäß Anspruch 6, wobei das Element (12) eine zentrale Längsachse aufweist, und wobei der Fingergriffring (34) befähigt ist, einer darauf ausgeübten Kraft in eine parallel zu der zentralen Längsachse verlaufenden Richtung von wenigstens 250 Gramm standzuhalten.

10. Applikator (10) gemäß Anspruch 9, wobei das dünnwandige Element (12) eine innere Lage (24) und eine äußere Lage (22) umfasst, und wobei die innere Lage (24) befähigt ist, um wenigstens etwa 4 Prozent mehr gedehnt zu werden als die äußere Lage (22).

11. Applikator (10) gemäß Anspruch 10, wobei die innere Lage (24) mit der äußeren Lage (22) durch ein Klebemittel verbunden ist.

12. Applikator (10) gemäß Anspruch 9, wobei der Fingergriffring (34) aus zwischen etwa 70 Prozent und etwa 95 Prozent Material und aus zwischen etwa 5 Prozent und etwa 30 Prozent Hohlraumfläche besteht.

13. Applikator (10) gemäß Anspruch 9, wobei der Fingergriffring (34) aus zwischen etwa 80 Prozent und etwa 90 Prozent Material und aus zwischen etwa 10 Prozent und etwa 20 Prozent Hohlraumfläche besteht.

14. Applikator (10) gemäß Anspruch 9, wobei der Fingergriffring (34) eine c-förmige Querschnittsanordnung aufweist.

15. Applikator (10) gemäß Anspruch 9, wobei der Fingergriffring (34) eine e-förmige Querschnittsanordnung aufweist.

16. Applikator (10) gemäß Anspruch 9, wobei der Fingergriffring (34) eine s-förmige Querschnittsanordnung aufweist.

17. Applikator (10) gemäß Anspruch 1, wobei das Element ein erstes Element (66) ist, das zum Halten und Abgeben eines Tampons vorgesehen ist, und wobei der Applikator (10) zusätzlich ein zweites Element (68) umfasst, das teleskopisch innerhalb des ersten Elements (66) verschiebbar ist, wobei das zweite Element (68) geeignet ist, das Tampon auszustoßen, wenn es in das erste Element (66) geschoben wird.

18. Applikator (10) gemäß Anspruch 17, wobei das erste Element (66) einen Außendurchmesser von weniger als etwa 2,54 cm (etwa 1 Inch) aufweist.

19. Applikator (10) gemäß Anspruch 18, wobei das erste Element (66) einen Außendurchmesser von weniger als etwa 1,9 cm (etwa 0,75 Inch) aufweist.

**20.** Applikator (10) gemäß Anspruch 17, wobei das erste Element (66) aus wenigstens drei separaten und unterschiedlichen Lagen gebildet ist.

**21.** Applikator (10) gemäß Anspruch 17, wobei eine der Lagen aus Papier ist.

**Revendications**

**1.** Applicateur (10) comprenant un élément creux (12) à paroi mince, adapté à retenir et distribuer une substance (14), ledit élément (12) étant formé à partir d'au moins deux couches séparées distinctes et ayant une première et une seconde extrémités espacées (26, 28) et un anneau de préhension au doigt (34) s'étendant vers l'extérieur, formé d'un seul tenant et adjacent à ladite seconde extrémité,

**caractérisé en ce que**

lesdites deux couches comprennent une couche intérieure (24) et une couche extérieure (22), et ladite couche intérieure (24) est capable de s'étirer davantage que ladite couche extérieure (22) de telle sorte que la couche intérieure (24) forme une boucle, la couche intérieure (24) formant la surface extérieure de l'anneau de préhension au doigt (34).

**2.** Applicateur (10) selon la revendication 1, dans lequel ledit anneau de préhension au doigt (34) a une densité qui est inférieure à celle dudit élément à paroi (12).

**3.** Applicateur (10) selon la revendication 2, dans lequel ledit anneau de préhension au doigt (34) a une densité qui est inférieure d'une valeur allant jusqu'à environ 20% à la densité dudit élément à paroi.

**4.** Applicateur (10) selon la revendication 1, dans lequel ledit anneau de préhension au doigt (34) est constitué d'environ 70% à environ 95% de matériau et d'environ 5% à environ 30% de vide.

**5.** Applicateur (10) selon la revendication 1, dans lequel ledit anneau de préhension au doigt (34) est constitué d'environ 80% à environ 90% de matériau et d'environ 10% à environ 20% de vide.

**6.** Applicateur (10) selon la revendication 1, dans lequel ledit anneau de préhension au doigt (34) est formé en roulant ladite seconde extrémité (28) dudit applicateur (10) sur elle-même sur au moins 180 degrés.

**7.** Applicateur (10) selon la revendication 6, dans lequel ledit anneau de préhension au doigt (34) est formé en roulant ladite seconde extrémité (28) dudit applicateur (10) sur elle-même sur au moins 270 degrés.

**8.** Applicateur (10) selon la revendication 6, dans lequel ledit anneau de préhension au doigt (34) est formé en roulant ladite seconde extrémité (28) dudit applicateur (10) sur elle-même sur au moins 360 degrés.

**9.** Applicateur (10) selon la revendication 6, dans lequel ledit élément (12) a un axe longitudinal central et dans lequel ledit anneau de préhension au doigt (34) est capable de résister à une force d'au moins 250 grammes appliquée sur lui selon une direction parallèle audit axe longitudinal central.

**10.** Applicateur (10) selon la revendication 9, dans lequel ledit élément (12) à paroi mince contient une couche intérieure (24) et une couche extérieure (22), et ladite couche intérieure (24) est capable de s'étirer d'au moins environ 4% de plus que ladite couche extérieure (22).

**11.** Applicateur (10) selon la revendication 10, dans lequel ladite couche intérieure (24) est liée à ladite couche extérieure (22) par un adhésif.

**12.** Applicateur (10) selon la revendication 9, dans lequel ledit anneau de préhension au doigt (34) est constitué d'environ 70% à environ 95% de matériau et d'environ 5% à environ 30% de vide.

**13.** Applicateur (10) selon la revendication 9, dans lequel ledit anneau de préhension au doigt (34) est constitué d'environ 80% à environ 90% de matériau et d'environ 10% à environ 20% de vide.

**14.** Applicateur (10) selon la revendication 9, dans lequel ledit anneau de préhension au doigt (34) a, en coupe transversale, une configuration en forme de c.

**15.** Applicateur (10) selon la revendication 9, dans lequel ledit anneau de préhension au doigt (34) a, en coupe transversale, une configuration en forme de e.

**16.** Applicateur (10) selon la revendication 9, dans lequel ledit anneau de préhension au doigt (34) a, en coupe transversale, une configuration en forme de s.

**17.** Applicateur (10) selon la revendication 1, dans lequel l'élément est un premier élément (66) adapté à retenir et distribuer un tampon, et dans lequel l'applicateur (10) comprend, en outre, un second élément (68) coulissant télescopiquement au sein dudit premier élément (66), ledit second élément (68) étant adapté à expulser ledit tampon tandis qu'il se déplace dans ledit premier élément (66).

**18.** Applicateur (10) selon la revendication 17, dans lequel ledit premier élément (66) a un diamètre extérieur inférieur à environ 2,54 cm (environ 1 pouce).

**19.** Applicateur (10) selon la revendication 18, dans lequel ledit premier élément (66) a un diamètre extérieur inférieur à environ 1,9 cm (environ 0,75 pouce).

**20.** Applicateur (10) selon la revendication 17, dans lequel ledit premier élément (66) est formé d'au moins trois couches séparées et distinctes.

**21.** Applicateur (10) selon la revendication 17, dans lequel l'une desdites couches est du papier.

FIG. 1

FIG. 2

FIG. 3

FIG. 4

FIG. 5

FIG. 6

FIG. 7

FIG. 8

17

**FIG. 9**

**FIG. 10**

EP 0 800 374 B2

FIG. 11          FIG. 12

FIG. 13

FIG. 14

FIG. 15

FIG. 17

FIG. 16

FIG. 18

FIG. 19

FIG. 20